(19) Europäisches Patentamt
European Patent Office
Office européen des brevets

(11) Veröffentlichungsnummer: **0 011 282**
**B1**

(12) # EUROPÄISCHE PATENTSCHRIFT

(45) Veröffentlichungstag der Patentschrift: **16.02.83**

(21) Anmeldenummer: **79104478.7**

(22) Anmeldetag: **13.11.79**

(51) Int. Cl.[3]: **C 07 D 333/24**, C 07 D 409/12, A 61 K 31/38

(54) **Thienylbenzoesäurederivate, Verfahren zu ihrer Herstellung und pharmazeutische Präparate auf Basis dieser Verbindungen.**

(30) Priorität: **16.11.78 DE 2849646**

(43) Veröffentlichungstag der Anmeldung:
**28.05.80 Patentblatt 80/11**

(45) Bekanntmachung des Hinweises auf die Patenterteilung:
**16.02.83 Patentblatt 83/7**

(84) Benannte Vertragsstaaten:
**AT BE CH DE FR GB IT NL**

(56) Entgegenhaltungen:
**DE - A - 2 654 795**

(73) Patentinhaber: **HOECHST AKTIENGESELLSCHAFT**
**Postfach 80 03 20**
**D-6230 Frankfurt/Main 80 (DE)**

(72) Erfinder: **Merkel, Wulf, Dr.**
**Hattersheimer Strasse 14**
**D-6233 Kelkheim (Taunus) (DE)**
Erfinder: **Bormann, Dieter, Dr.**
**Johann-Strauss-Strasse 20**
**D-6233 Kelkheim (Taunus) (DE)**
Erfinder: **Mania, Dieter, Dr.**
**Berliner Ring 5**
**D-6233 Kelkheim (Taunus) (DE)**
Erfinder: **Muschaweck, Roman, Dr.**
**Heimchenweg 39**
**D-6230 Frankfurt am Main 80 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 282 B1

Thienylbenzoesäurederivate, Verfahren zu ihrer Herstellung und pharmazeutische Präparate auf Basis dieser Verbindungen

Gegenstand der Erfindung sind Thienylbenzoesäurederivate der allgemeinen Formel I

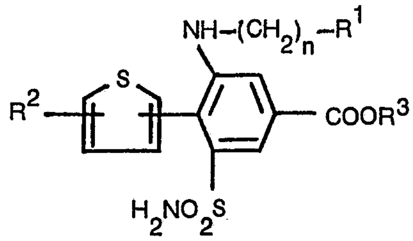

(I)

in der $R^1$ Phenyl, 2- 3- oder 4-Fluorphenyl, 2- oder 3-Thienyl, 2- oder 3-Furyl, $R^2$ Wasserstoff oder Methyl und $R^3$ Wasserstoff, Alkyl mit 1—4 Kohlenstoffatomen oder Benzyl bedeuten und n für die Zahl 1 oder 2 steht sowie deren pharmazeutisch verträglichen Salze mit Säuren oder Basen.

Gegenstand der Erfindung ist weiterhin ein Verfahren zur Herstellung der Verbindungen der Formel I, das dadurch gekennzeichnet ist, daß man Verbindungen der Formel II

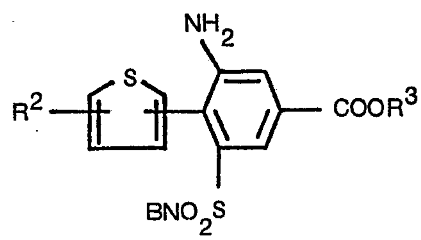

(II)

worin $R^2$ und $R^3$ die obige Bedeutung haben und B für 2 Wasserstoffatome oder die Gruppe $(CH_3)_2N—CH=$ steht, mit Verbindungen der Formel III

$$R^1—(CH_2)_n—Y \qquad (III)$$

worin $R^1$ und n obige Bedeutung haben und Y eine leaving-group bedeutet, umsetzt oder mit Aldehyden der Formel IV

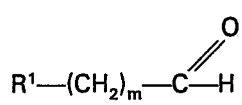

(IV)

worin m für die Zahl 0 oder 1 steht, kondensiert und die erhaltenen Azomethine entweder gleichzeitig oder anschliessend reduziert oder
Verbindungen der Formel V

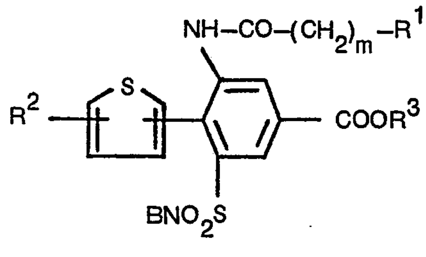

(V)

worin $R^1$ bis $R^3$, B, und m obige Bedeutung haben, mit Diboran oder komplexen Borhydriden gegebenenfalls in Gegenwart von Lewis Säuren reduziert und die so erhaltenen Verbindungen gegebenenfalls anschließend hydrolisiert und gegebenenfalls durch Behandlung mit pharmazeutisch verträglichen Säuren oder Basen in ihre Salze überführt.

Von besonderer Bedeutung sind Verbindungen der Formel I, in der n für 1 steht.

Das erfindungsgemäße Verfahren betrifft die Monosubstitution der Verbindungen der allgemeinen Formel II nach an sich üblichen Methoden. Bei der Alkylierung der Verbindungen II mit Alkylierungsmitteln der Formel III kommt als "leaving-group" Y insbesondere Halogen, vorzugsweise Brom, eine Hydroxy- oder Sulfonyloxy-, Alkyl- oder Arylsulfonyloxygruppe in Betracht. Die Umsetzung erfolgt gegebenenfalls in Gegenwart einer Base zur Bindung der freiwerdenden Säure, wobei man vorzugsweise organische tertiäre Amine wie Trimethylamin, Triäthylamin oder Pyridin verwendet. Als anorganische Basen kommen vorzugsweise Natriumbicarbonat oder Kalliumcarbonat in Betracht.

Bei der Monosubstitution via Azomethine werden Aldehyde der Formel IV eingesetzt, wobei man die üblichen Verfahren der Azomethin (Schiff-Base) Bildung verwendet. Die Reduktion der erhaltenen Azomethine erfolgt enweder gleichzeitig, indem man die Aldehyde der Formel IV in Gegenwart der Amine der Formel II katalytisch hydriert oder nach erfolgter Kondensation in üblicher Weise, beispielsweise mit Natriumborhydrid reduziert. Hierbei verwendet man die für solche Reduktionen üblichen Lösungsmittel, z.B. niedere aliphatische Alkohole.

Die Herstellung der Verbindung der Formel I durch Reduktion der Amido-Verbindungen der Formel V kann analog den in DOS 2 345 229 bzw. 2 453 548 beschriebenen Verfahrensweisen durchgeführt werden. Man reduziert entweder mit Diboran oder komplexen Borhydriden in Gegenwart von Lewis-Säuren oder mit Diboran allein; besonders vorteilhaft ist die Kombination Natriumborhydrid/Bortrifluorid. Die Reaktions-bedingungen sind ausführlich in den vorgenannten Deutschen Offenlegungsschriften beschrieben.

Bei allen Verfahrensvarianten ist es von besonderem Vorteil, die Sulfamylgruppe durch die Schutzgruppe B zu schützen, dahierdurch Nebenreaktionen an der Sulfonamidgruppe verhindert werden. Nach erfolgter Substitution der Aminogruppe kann die Schutzgruppe B durch basische oder saure Hydrolyse abgespalten werden, wobei gegebenenfalls gleichzeitig eine etwa vorhandene Estergruppe $COOR^3$ in die freie Carboxylgruppe überführt wird.

Die Ausgangsstoffe der allgemeinen Formel II sind aus DOS 2 654 795 bekannt bzw. können in analoger Weise hierzu hergestellt werden. Will man von Verbindungen der Formel II ausgehen, in der B für 2 Wasserstoffatome steht, läßt sich die Schutzgruppe aus den vorbekannten Verbindungen durch die alkalische oder saure Hydrolyse abspalten.

Von Seite 9 der DOS 26 54 795 ist bekannt, daß N,N-Dialkylaminomethylenamino-sulfonylbenzoesäure-Derivate als Zwischenprodukte zur Synthese von Arzneimitteln, wie Diuretika und Saluretika dienen können.

Die Herstellung der Ausgangsstoffe der Formel V folgt analog den in DOS 2 345 229 und 2 453 548 beschriebenen Acylierungsverfahren. Die Amine der Formel II werden mit zur Amidbildung geeigneten Carbonsäurederivaten, insbesondere Säurechloriden oder Carbonsäureestern unter den in den genannten Offenlegungsschriften beschriebenen Reaktionsbedingungen in die Amidoverbindungen der Formel V überführt.

Die nach den obigen Verfahren erhaltenen endprodukte der Formel I können mit pharmazeutisch verträglichen Säuren wie mit pharmazeutisch verträglichen Basen Salze bilden. Hierfür kommen die für Pharmazeutika üblichen Säuren bzw. Basen in Betracht.

Die Sulfamoylbenzoesäurederivate der Formel I sowie ihre pharmazeutisch verträglichen Salze sind hochwirksame Diuretika und Saluretika, die als Pharmazeutika in Human- und Veterinärmedizin eingesetzt werden können.

Sie werden in Dosierungen von 0,5 bis 100 mg in Kapeln, Dragees, Tabletten oder Lösungen mit verschiedenen Zusätzen enteral z.B. oral mit Sonde oder parenteral (Injektion in das Gefäßsystem, z.B. intravenös oder auch Injektion in die Muskulatur oder unter die Haut) verabfolgt. Sie eignen sich zur Behandlung von Ödemkrankheiten, wie kardiale, renale oder hepatisch bedingter Ödeme und anderer derartiger auf Störungen des Wasser- und Elektrolyt-Haushalts zurückzuführenden Erscheinungen. Die Verbindungen können allein oder in Kombination mit anderen salidiuretisch wirksamen Substanzen auch anderer Wirkungsart angewendet werden, oder mit verschiedenen anderen Arzneimitteln getrennt, alternierend oder in Kombination verabfolgt werden. Insbesondere sind zu nennen SPIRONOLACTON, TRIAMTEREN, AMILORID und andere $K^+$-retinierende Verbindungen alternierend mit langwirkenden Salidiuretika vom Typ des CHLORTHALIDONS oder anderen $K^+$-Verlust substituierenden Kalium-enthaltenden Verbindungen (Salze).

Außer den in den Beispielen beschriebenen Verbindungen sind die nachstehend aufgeführten erfindungsgemäßen Verbindungen von besonderem Interesse:

3-(4-Fluorbenzylamino)-4-(2-thienyl)-5-sulfamoyl-benzoesäure
3-(4-Fluorbenzylamino)-4-(3-thienyl)-5-sulfamoyl-benzoesäure
3-(3-Fluorbenzylamino)-4-(2-thienyl)-5-sulfamoyl-benzoesäure
3-(3-Fluorbenzylamino)-4-(3-thienyl)-5-sulfamoyl-benzoesäure
3-(2-Fluorbenzylamino)-4-(2-thienyl)-5-sulfamoyl-benzoesäure
3-(2-Fluorbenzylamino)-4-(3-thienyl)-5-sulfamoyl-benzoesäure
3-(3-Thenylamino)-4-(5-methyl-2-thienyl)-5-sulfamoyl-benzoesäure
3-(3-Thenylamino)-4-(5-methyl-3-thienyl)-5-sulfamoyl-benzoesäure
3-(2-Thenylamino)-4-(5-methyl-2-thienyl)-5-sulfamoyl-benzoesäure
3-(2-Thenylamino)-4-(5-methyl-3-thienyl)-5-sulfamoyl-benzoesäure
3-(2-Furylmethylamino)-4-(2-thienyl)-5-sulfamoyl-benzoesäure
3-(2-Furylmethylamino)-4-(3-thienyl)-5-sulfamoyl-benzoesäure

Beispiel 1

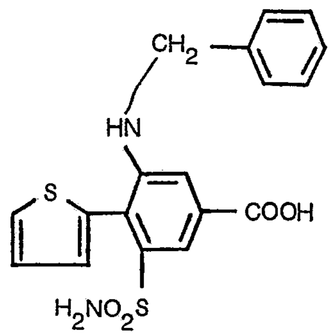

3-Benzylamino-4-(2-thienyl)-5-sulfamoyl-benzoesäure

a) *3-Benzylamino-4-(2-thienyl)-5-N,N-dimethylaminomethylenamino-sulfonyl-benzoesäuremethylester*

11 g 3 - Amino - 4 - (2 - thienyl) - 5 - N,N - dimethylaminomethylenamino - sulfonyl - benzoesäuremethylester werden in 100 ml Dimethylformamid gelöst und 5 g Benzylbromid zugegeben. Man erhitzt zum Rückfluß und tropft 2 ml Triäthylamin hinzu. Nach 4 bis 5 Stunden läßt man das Reaktionsgemisch erkalten und rührt es in 500 ml Eiswasser ein. Das ausgefallene Produkt wird aus $CH_3OH$ oder Äthanol kristallisiert.

*Schmp.: 163—165°C.*

b) 9,4 g des bei (1a) erhaltenen Esters werden in 2 n NaOH suspendiert und bis zur klaren Lösung am Rückfluß erhitzt. Danach fällt man die freie Säure mit 2 n HCl in der Kälte aus und kristallisiert das Produkt aus Eisessig oder $CH_3OH/H_2O$ um.

Hellgelbe Nadeln vom *Schmp.: 243—246°C*

NMR-Daten: ($D_6$-DMSO, 60 MHz, TMS) in ppm: $\delta = 4,4$ (d, 2H), $\delta = 5,25$ (t, 1H), $\delta = 6,8—7,5$ (m, 10H), $\delta = 7,66—8,0$ (m, 2H)

Beispiel 2

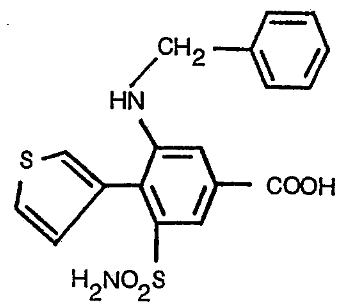

3-Benzylamino-4-(3-thienyl)-5-sulfamoyl-benzoesäure

a) *3-Benzylamino-4-(3-thienyl-5-N,N-dimethylaminomethylenamino-sulfonyl-benzoesäuremethylester*

5,5 g 3 - Amino - 4 - (3 - thienyl) - 5 - N,N-dimethylaminomethylenamino - sulfonyl - benzoesäuremethylester werden in 50 ml Dimethylformamid gelöst, 3,5 ml Benzylbromid und 1,5 ml Triäthylamin zugegeben und 1,5 Stunden am Rückfluß erhitzt. Danach tropft man die Lösung in Eiswasser ein, isoliert das Produkt und kristallisiert es aus $CH_3OH/H_2O$ oder $CH_3OH$ um.

*Schmp. 139—140°C.*

b) 4,9 g des bei (2a) erhaltenen Esters werden in 2 n NaOH suspendiert und bis zur klaren Lösung am Rückfluß erhitzt. Danach fällt man das Produkt mit 2 n HCl in der Kälte aus und kristallisiert es aus $CH_3OH/H_2O$ oder Eisessig um.

Hellgelbe Nadeln vom *Schmp.: 245—247°C*

NMR-Daten: ($D_6$-DMSO, 60 MHz, TMS) in ppm: $\delta = 4,3$ (d, 2H), $\delta = 4,9$ (t, 1H) $\delta = 6,5—7,8$ (m, 12H)

Beispiel 3

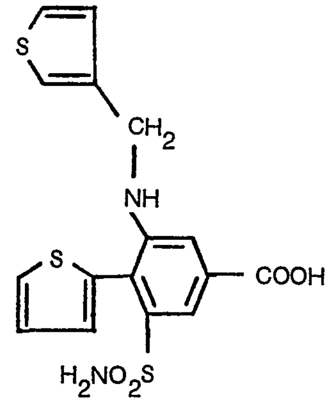

3-(3'-Thenylamino)-4-(2-thienyl)-5-sulfamoyl-benzoesäure

18,4 g 3 - Amino - 4 - (2 - thienyl) - 5 - N,N - dimethylaminomethylenamino - sulfonyl - benzoesäuremethylester werden in 100 ml abs. Dimethylformamid gelöst und zum Rückfluß erhitzt. Dann tropft man gleichzeitig und unter starkem Rühren jeweils 35 g 3-Thenylbromid und 15 g Triäthylamin getrennt hinzu. Nach 2 Stunden wird die Mischung eingeengt und das zurückbleibende Öl mehrmals mit Petroläther und danach mit Wasser digeriert.

Die zurückbleibende feste Masse wird bis zur klaren Lösung mit 2 n NaOH auf dem Dampfbad erwärmt. Danach wird filtriert und mit 2 n HCl auf pH 3—4 eingestellt. Das ausgefallene Produkt kann aus $CH_3OH$, Eisessig oder Acetonitril umkristallisiert werden. Hellgelbe Kristalle vom Schmp. 206—207°C.

NMR-Daten: ($D_6$-DMSO, 60 MHz, TMS) in ppm: $\delta = 4{,}31$ (d, 2H), $\delta = 5{,}06$ (t, 1H), $\delta = 6{,}7$—$8{,}0$ (m, 10H)

Beispiel 4

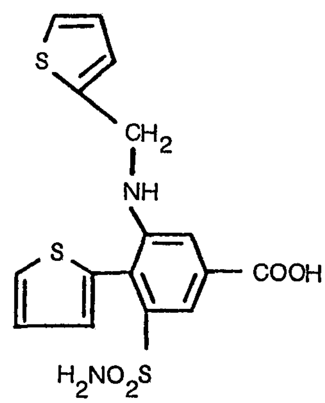

3-(2'-Thenylamino)-4-(2-thienyl)-5-sulfamoyl-benzoesäure

a) *3-(2'-Thenoylamino)-4-(2-thienyl)-5-N,N-dimethylaminomethylen-amino-sulfonyl-benzoesäuremethylester*

Zur siedenden Lösung von 0,037 Mol (~13,5 g) 3-Amino-4-(2-thienyl)-5-N,N-dimethylaminomethylenaminosulfonyl-benzoesäuremethylester und 0,045 Mol (~3,6 ml) Pyridin in 100 ml abs. Dioxan tropft man langsam unter Rühren eine Lösung von 0,074 Mol (7,9 ml) 2-Thenoylchlorid in Aceton hinzu. Das Produkt kristallisiert schon teilweise während der Reaktion aus. Nach 2 Stunden wird abfiltriert und das Produkt gut mit Äther gewaschen. Schmp. 233—234°C.

b) 15,9 g (0,033 Mol) Amid (Beispiel 4a) werden in 450 ml abs. Diglyme suspendiert und 9,5 ml Bortrifluorid-Ätherat zugegeben. Dann tropft man bei 50°C unter gutem Rühren eine Lösung von 1,8 g Natriumborhydrid in 150 ml abs. Diglyme hinzu und rührt bei 50—70°C zwei Stunden nach. Das Produkt wird schließlich mit Eiswasser ausgefällt, isoliert und mit 2 n NaOH bis zur klaren Lösung auf dem Dampfbad verseift. Bei Zugabe von 2 n HCl bis pH 3—4 fällt die 3-(2'-Thenylamino)-4-(2-thienyl)-5-sulfamoyl-benzoesäure aus. Umkristallisation aus $CH_3OH$ oder $CH_3OH/H_2O$

Schmp. 115—117°C (hochviskose Schmelze)

NMR-Daten: ($D_6$-DMSO, 60 MHz, TMS) in ppm: $\delta = 4{,}5$ (d, 2H), $\delta = 5{,}2$ (t, 1H), $\delta = 6{,}7$—$7{,}5$ (m, 8H), $\delta = 7{,}5$—$8$ (m, 2H)

Beispiel 5

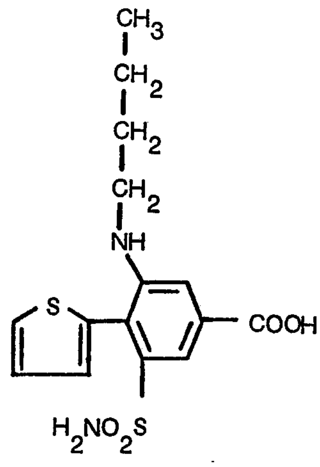

4-(2-Thienyl-3-N-butylamino-5-sulfamoyl-benzoesäure

a) *3-Butyrylamino-4-(2-thienyl)-5-N,N-dimethylaminomethylen-aminosulfonyl-benzoesäuremethylester*

11,2 g (0,03 Mol) 3 - Amino - 4 - (2 - thienyl) - 5 - N,N - dimethylaminomethylenaminosulfonyl - benzoesäuremethylester werden in 100 ml abs. Dioxan und 3 ml Pyridin zum Rückfluß erhitzt. Dazu tropft man 6,4 g Buttersäurechlorid in 50 ml Aceton gelöst hinzu. Nach 1/2 Stunde wird das Amid mit Eiswasser ausgefällt und aus $CH_3OH$ umkristallisiert.
Schmp. 202—203°C.

b) 8,1 g Amid werden in 150 ml abs. Diglyme gelöst, 4,7 ml $BF_3$-Ätherat zugegeben und auf 50°C wenig $H_2O$ zersetzt und schließlich mit Eiswasser gefällt. Das ausgefallene Rohprodukt wird mit 2 n NaOH bis zur klaren Lösung auf dem Dampfbad verseift und die freie Säure dann mit HCl bei pH 3—4 ausgefallt. Umkrist. aus $CH_3OH/H_2O$ Schmp. 157—158°C

NMR-Daten: ($D_6$-DMSO, 60 MHz, TMS) in ppm: $\delta = 0{,}5$—1,67 (m, 7H) $\delta = 3{,}06$ (m, 2H) $\delta = 4{,}25$ (t, 1H) $\delta = 6{,}7$—7,4 (m, 5H) $\delta = 7{,}6$—7,9 (m, 2H)

Beispiel 6

3-Furfurylamino-4-(2-thienyl)-5-sulfamoylbenzoesäure

a) Zu einer Mischung von 7,34 g (0,02 Mol) 3 - Amino - 4 - (2 - thienyl) - 5 - N,N - dimethylaminomethylenaminosulfonyl - benzoesäuremethylester und 1,5 g $K_2CO_3$ in 75 ml Aceton werden bei Raumtemperatur eine Lösung von 2,86 g Furan-2-carbonsäurechlorid in 10 ml Aceton zugefügt und die Reaktionsmischung 90 Min. zum Sieden erhitzt. Nach dem Abkühlen wird mit 50 ml Wasser versetzt, 30 Minuten unter Eiskühlung nachgerührt, wobei der 3 - (2 - Furfuroylamido) - 4 - (2 - thienyl) - 5 - N,N - dimethylaminomethylenaminosulfonylbenzoesäuremethylester als cremefarbene Kristalle vom Schmp. 216—218° ausfällt, der nach guter Trocknung ohne weitere Reinigung direkt weiter umgesetzt wird.

b) Zu einer Mischung aus 2 g der obigen Amidoverbindung in 60 ml Diglyme werden bei Raumtemperatur 1,6 ml $BF_3$-ätherat hinzugegeben, 15 Minuten nachgerührt und anschließend die Suspension mit einer Lösung von 500 mg $NaBB_4$ in 50 ml Diglyme tropfenweise versetzt. Nach 60 Minuten bei Raumtemperatur wird 60 Minuten bei 60° nachgerührt, die Reaktionsmischung gekühlt und zunächst vorsichtig mit Wasser hydrolysiert. Nach Ende der Wasserstoffentwicklung wird die Reaktionsmischung in 500 ml Wasser eingegossen, das ausgefallene Produkt mit Essigester extrahiert und nach dem Trocknen der organischen Phase mit $MgSO_4$ das Lösungsmittel entfernt.

Der zurückbleibende Rückstand wird aus Äthanol umkristallisiert. Man isoliert den 3 - (2 - Furfurylamino) - 4 - (2 - thienyl) - 5 - N,N - dimethylaminosulfonylbenzoesäuremethylester als cremefarbene Kristalle vom Schmp. 190—192°.

c) Eine Mischung aus 8 g des so erhaltenen 3- (2 - Furfurylamino) - 4 - (2 - thienyl) - 5 - N,N - dimethylaminosulfonylbenzoesäuremethylesters in 200 ml 1n NaOH werden 60 Minuten auf dem Dampfbad erhitzt, wobei sich eine klare Lösung bildet. Nach weiteren 30 Min. auf dem Dampfbad wird filtriert, gekühlt und anschließend mit 2 n HCl angesäuert. Der ausgefallene Feststoff wird isoliert und aus Methanol/Wasser 1:1 umkristallisiert. Man isoliert die 3 - (2 - Furfurylamino) - 4 - (2 - thienyl) - 5 - sulfamylbenzoesäure als cremefarbene Kristalle vom Schmp. 97—103°.

NMR-Daten: ($D_6$-DMSO, 60 MHz, TMS) in ppm: $\delta = 4{,}4$ (d, 2H), $\delta = 5{,}0$ (t, 1H) $\delta = 6{,}13$ (m, 1H) $\delta = 6{,}33$ (m, 1H) $\delta = 6{,}9$—$8{,}0$ (m, 8H)

**Patentansprüche für die Vertragsstaaten: BE CH DE FR GB IT NL**

1. Thienylbenzoesäurederivate der allgemeinen Formel I

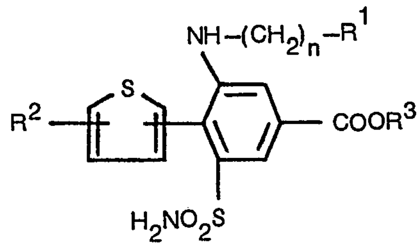

(I)

in der $R^1$ Phenyl, 2-, 3- oder 4-Fluorphenyl, 2- oder 3-Thienyl, 2- oder 3-Furyl, $R^2$ Wasserstoff oder Methyl und $R^3$ Wasserstoff, Alkyl mit 1—4 Kohlenstoffatomen oder Benzyl bedeuten und n für die Zahl 1 oder 2 steht sowie deren pharmazeutisch verträglichen Salze mit Säuren oder Basen.

2. Verbindungen der allgemeinen Formel I gemäß Anspruch 1 in der n für die Zahl 1 steht.

3. 3-Benzylamino-4-(2-thienyl)-5-sulfamoyl-benzoesäure

4. 3-Benzylamino-4-(3-thienyl)-5-sulfamoyl-benzoesäure

5. 3-(3′-Thenylamino)-4-(2-thienyl)-5-sulfamoyl-benzoesäure

6. 3-(2′-Thenylamino)-4-(2-thienyl)-5-sulfamoyl-benzoesäure

7. Verfahren zur Herstellung der Verbindungen der Formel I, dadurch gekennzeichnet, daß man Verbindungen der Formel II

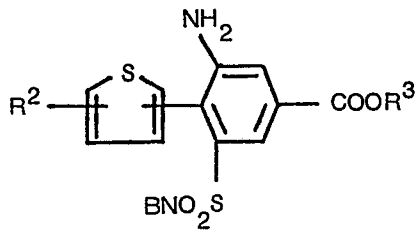

(II)

worin $R^2$ und $R^3$ die obige Bedeutung haben und B für 2 Wasserstoffatome oder die Gruppe $(CH_3)_2N$—$CH=$ steht, mit Verbindungen der Formel III

$$R^1—(CH_2)_n—Y \qquad (III)$$

worin $R^1$ und n obige Bedeutung haben und Y eine leaving-group bedeutet, umsetzt oder mit Aldehyden der Formel IV

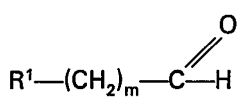

(IV)

worin m für die Zahl 0 oder 1 steht, kondensiert und die erhaltenen Azomethine entweder gleichzeitig oder anschließend reduziert oder
Verbindungen der Formel V

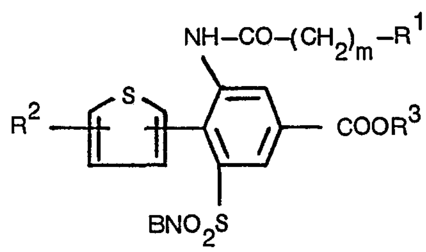

(V)

worin $R^1$ bis $R^3$, B, und m obige Bedeutung haben, mit Diboran oder komplexen Borhydriden gegebenenfalls in Gegenwart von Lewis Säuren reduziert und die so erhaltenen Verbindungen gegebenenfalls anschließend hydrolisiert und gegebenenfalls durch Behandlung mit pharmazeutisch verträglichen Säuren oder Basen in ihre Salze überführt.

8. Verfahren zur Herstellung von pharmazeutischen Präparaten, dadurch gekennzeichnet, daß man eine Verbindung gemäß Anspruch 1, gegebenenfalls zusammen mit pharmazeutisch üblichen Trägerstoffen und/oder Stabilisatoren in eine für therapeutische Zwecke geeignete Anwendungsform bringt.

9. Pharmazeutische Präparate, bestehend aus oder enthaltend Verbindungen der allgemeinen Formel I gemäß Anspruch 1.

**Patentansprüche für den Vertragsstaat: AT**

1. Verfahren zur Herstellung von Thienylbenzoesäurederivaten der allgemeinen Formel I

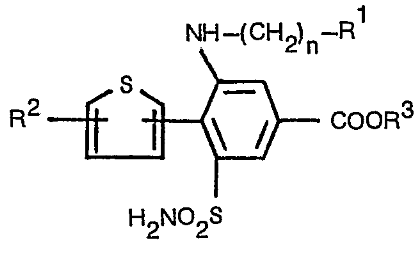

(I)

in der $R^1$ Phenyl, 2-, 3- oder 4-Fluorphenyl, 2- oder 3-Thienyl, 2- oder 3-Furyl, $R^2$ Wasserstoff oder Methyl und $R^3$ Wasserstoff, Alkyl mit 1—4 Kohlenstoffatomen oder Benzyl bedeuten und n für die Zahl 1 oder 2 steht sowie deren pharmazeutisch verträglichen Salze mit Säuren oder Basen, dadurch gekennzeichnet, daß man Verbindungen der Formel II

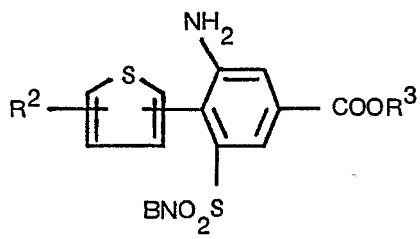

(II)

worin $R^2$ und $R^3$ die obige Bedeutung haben und B für 2 Wasserstoffatome oder die Gruppe $(CH_3)_2N—CH=$ steht, mit Verbindungen der Formel III

$$R^1—(CH_2)_n—Y \qquad (III)$$

worin $R^1$ und n obige Bedeutung haben und Y eine leaving-group bedeutet, umsetzt oder mit Aldehyden der Formel IV

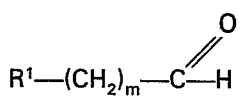

(IV)

worin m für die Zahl 0 oder 1 steht, kondensiert und die erhaltenen Azomethine entweder gleichzeitig oder anschließend reduziert oder

Verbindungen der Formel V

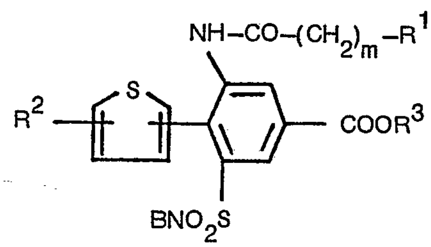

(V)

worin $R^1$ bis $R^3$, B, und m obige Bedeutung haben, mit Diboran oder komplexen Borhydriden gegebenenfalls in Gegenwart von Lewis Säuren reduziert und die so erhaltenen Verbindungen gegebenenfalls anschließend hydrolisiert und gegebenenfalls durch Behandlung mit pharmazeutisch verträglichen Säuren oder Basen in ihre Salze überführt.

2. Verfahren gemäß Anspruch 1, dadurch gekennzeichnet, daß in der allgemeinen Formel I n für die Zahl 1 und in den allgemeinen Formeln IV und V m für O stehen.

3. Verfahren gemäß Anspruch 1, dadurch gekennzeichnet, daß man die 3-Benzylamino-4-(2-thienyl)-5-sulfamoyl-benzoesäure herstellt.

4. Verfahren gemäß Anspruch 1, dadurch gekennzeichnet, daß man die 3-Benzylamino-4-(3-thienyl)-5-sulfamoyl-benzoesäure herstellt.

5. Verfahren gemäß Anspruch 1, dadurch gekennzeichnet, daß man die 3-(3′-Thenylamino)-4-(2-thienyl)-5-sulfamoylbenzoesäure herstellt.

6. Verfahren gemäß Anspruch 1, dadurch gekennzeichnet, daß man die 3-(2′-Thenylamino)-4-(2-thienyl)-5-sulfamoyl-benzoesäure herstellt.

**Claims for the Contracting states: BE CH DE FR GB IT NL**

1. Thienylbenzoic acid derivatives of the general formula

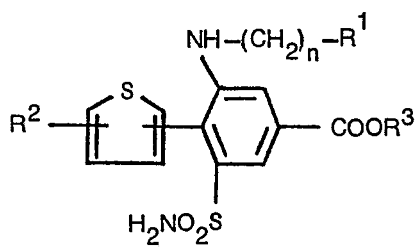

(I)

in which $R^1$ is phenyl, 2-, 3- or 4-fluorophenyl, 2- or 3-thienyl, 2- or 3-furyl; $R^2$ is hydrogen or methyl; $R^3$ is hydrogen or alkyl having from 1 to 4 carbon atoms or benzyl; and n is 1 or 2; and the pharmaceutically tolerable salts thereof with acids or bases.

2. Compounds of the general formula I as claimed in Claim 1, in which n is 1.

3. 3-Benzylamino-4-(2-thienyl)-5-sulfamoyl-benzoic acid.

4. 3-Benzylamino-4-(3-thienyl)-5-sulfamoyl-benzoic acid.

5. 3-(3′-Thenylamino)-4-(2-thienyl)-5-sulfamoyl-benzoic acid.

6. 3-(2′-Thenylamino)-4-(2-thienyl)-5-sulfamoyl-benzoic acid.

7. Process for the preparation of the compounds of formula I, which comprises reacting compounds of the formula II

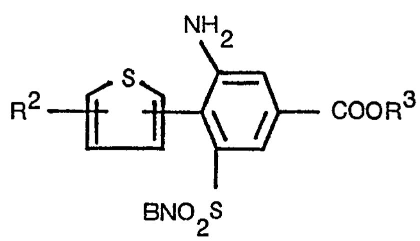

(II)

in which $R^2$ and $R^3$ are as defined above, and B represents 2 hydrogen atoms or the $(CH_3)_2N—CH{=}$group, with compounds of the formula III

$$R^1—(CH_2)_n—Y \qquad (III)$$

in which $R^1$ and n are as defined above and Y is a leaving-group; or condensing them with aldehydes of the formula IV

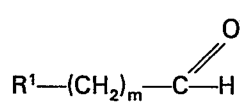

(IV)

in which m is zero or 1, and reducing the azomethines obtained either simultaneously or subsequently; or reducing compounds of the formula V

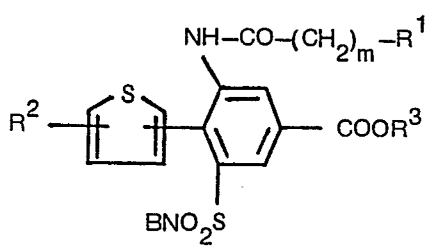

(V)

in which $R^1$ to $R^3$, B and m are as defined above, with diborane or complex boron hydrides, optionally in the presence of Lewis acids, and optionally hydrolizing thereafter the compounds so obtained, and optionally converting them to their salts by treatment with corresponding pharmaceutically tolerable acids or bases.

8. Process for the manufacture of pharmaceutical products, which comprises giving a compound as claimed in Claim 1, optionally together with pharmaceutically usual carriers and/or stabilizers, an application form suitable for therapeutical purposes.

9. Pharmaceutical products consisting of or containing compounds of the general formula I as claimed in Claim 1.

**Claims for the Contracting state: AT**

1. Process for the preparation of the thienylbenzoic acid derivatives of the general formula

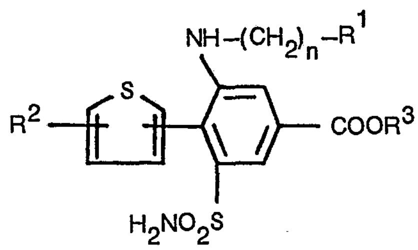

(I)

in which $R^1$ is phenyl, 2-, 3- or 4-fluorophenyl, 2- or 3-thienyl, 2- or 3-furyl; $R^2$ is hydrogen or methyl; $R^3$ is hydrogen or alkyl having from 1 to 4 carbon atoms or benzyl; and n is 1 or 2; and the pharmaceutically tolerable salts thereof with acids or bases, which comprises reacting compounds of the formula II

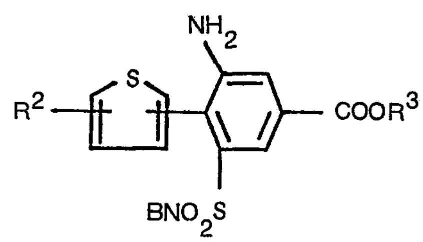

(II)

in which $R^2$ and $R^3$ are as defined above, and B represents 2 hydrogen atoms or the $(CH_3)_2N{-}CH{=}$group, with compounds of the formula III

$$R^1-(CH_2)_n-Y \qquad \text{(III)}$$

in which $R^1$ and n are as defined above and Y is a leaving-group; or condensing them with aldehydes of the formula IV

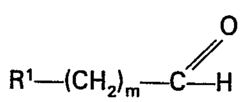

(IV)

in which m is zero or 1, and reducing the azomethines obtained either simultaneously or subsequently; or reducing compounds of the formula V

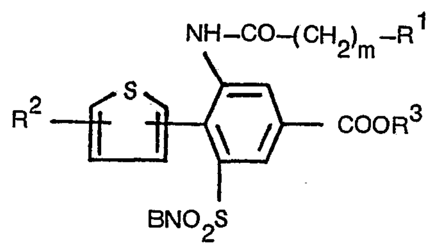

(V)

in which $R^1$ to $R^3$, B and m are as defined above, with diborane or complex boron hydrides, optionally in the presence of Lewis acids, and optionally hydrolizing thereafter the compounds so obtained, and optionally converting them to their salts by treatment with corresponding pharmaceutically tolerable acids or bases.

2. Process according to Claim 1, characterized in that in general formula I n is 1 and in general formulae IV and V m is 0.

3. Process according to Claim 1, characterized in that a compound is prepared which is 3-benzylamino-4-(2-thienyl)-5-sulfamoyl-benzoic acid.

4. Process according to claim 1, characterized in that a compound is prepared which is 3-benzylamino-4-(3-thienyl)-5-sulfamoyl-benzoic acid.

5. Process according to Claim 1, characterized in that a compound is prepared which is 3-(3′-thenylamino)-4-(2-thienyl)-5-sulfamoyl-benzoic acid.

6. Process according to Claim 1, characterized in that a compound is prepared which is 3-(2′-thenylamino)-4-(2-thienyl)-5-sulfamoyl-benzoic acid.

**Revendications pour les Etats contractants: BE CH DE FR GB IT NL**

1. Dérivés de l'acide thiénylbenzoïque répondant à la formule générale I

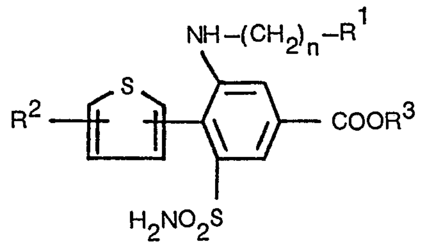

(I)

dans laquelle $R^1$ désigne un radical phényle, 2-, 3- ou 4-fluorophényle, 2- ou 3-thiényle, 2- ou 3- furyle, $R^2$ l'hydrogène ou un radical méthyle et $R^3$ l'hydrogène, un radical alcoyle en $C_1$ à $C_4$ ou benzyle et n représente les nombres 1 ou 2 ainsi que leurs sels avec des acides ou des bases pharmaceutiquement acceptables.

2. Composés répondant à la formule générale I suivant la revendication 1, dans laquelle désigne le nombre 1.

3. Acide 3-benzylamino-4-(2-thiényl)-5-sulfamoyl-benzoïque.

4. Acide 3-benzylamino-4-(3-thiényl)-5-sulfamoyl-benzoïque.

5. Acide 3-(3′-thénylamino)-4-(2-thiényl)-5-sulfamoyl-benzoïque.

6. Acide 3-(2′-thénylamino)-4-(2-thiényl)-5-sulfamoyl-benzoïque.

7. Procédé de préparation des composés répondant à la formule I caractérisé en ce qu'on condense des composés répondant à la formule II

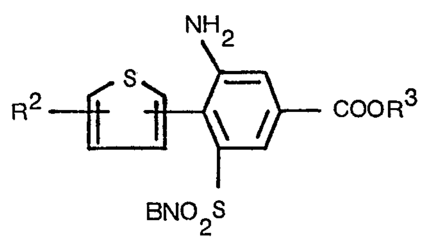

(II)

dans laquelle $R^2$ et $R^3$ ont la signification ci-dessus et B représente 2 atomes d'hydrogène ou le groupe $(CH_3)N{-}CH{=}$, avec des composés répondant à la formule III

$$R^1{-}(CH_2)_n{-}Y \qquad \text{(III)}$$

dans laquelle $R^1$ et n ont la signification ci-dessus et Y désigne un leaving-group, ou on condense avec des aldéhydes répondant à la formule IV

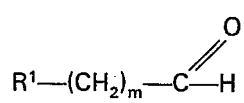

(IV)

où m désigne 0 ou 1, et on réduit simultanément ou ultérieurement les azométhines obtenues, ou on réduit des composés répondant à la formule V

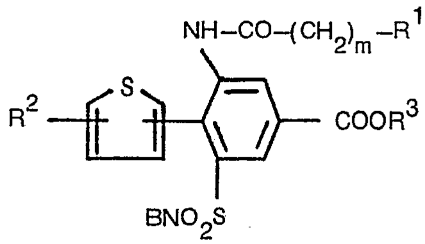

(V)

dans laquelle $R^1$ à $R^3$, B et m ont la signification ci-dessus, avec du diborane ou des borohydrures complexes, le cas échéant en présence d'acides de Lewis, et on hydrolyse ensuite le cas échéant les composés ainsi obtenus et on les transforme le cas échéant en leurs sels par traitement avec des acides ou des bases pharmaceutiquement acceptables.

8. Procédé de préparation de compositions pharmaceutiques, caractérisé en ce qu'on amène un composé suivant la revendication 1, le cas échéant avec des excipients et/ou des stabilisants habituels en pharmacie, sous une forme d'utilisation appropriée à des buts thérapeutiques.

9. Compositions pharmaceutiques constituées de, ou contenant des composés répondant à la formule générale I suivant la revendication 1.

**Revendications pour l'Etat contractant: AT**

1. Procédé de préparation de dérivés de l'acide thiénylbenzoïque repondant à la formule générale I

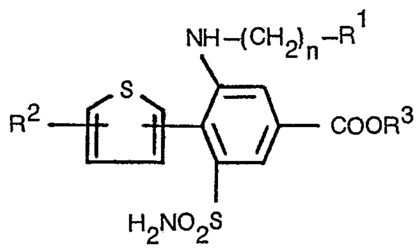

(I)

dans laquelle $R^1$ désigne un radical phényle, 2-, 3- ou 4-fluorophényle, 2- ou 3-thiényle, 2- ou 3- furyle, $R^2$ l'hydrogène ou un radical méthyle et $R^3$ l'hydrogène, un radical alcoyle en $C_1$ à $C_4$ ou benzyle et n représente les nombres 1 ou 2 ainsi que de leurs sels avec des acides ou des bases pharmaceutiquement acceptables caractérisé en ce qu'on condense des composés répondant à la formule II

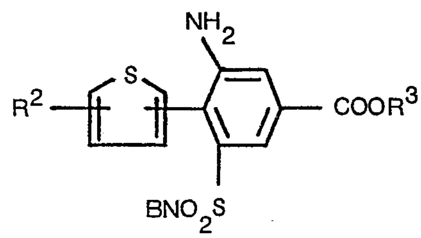

(II)

dans laquelle $R^2$ et $R^3$ ont la signification ci-dessus et B représente 2 atomes d'hydrogène ou le groupe $(CH_3)N—CH=$, avec des composés répondant à la formule III

$$R^1—(CH_2)_n—Y \quad \text{(III)}$$

dans laquelle $R^1$ et n ont la signification ci-dessus et Y désigne un leaving-group, ou on condense avec des aldéhydes répondant à la formule IV

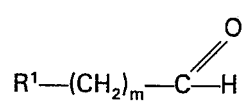

(IV)

où m désigne 0 ou 1, et on réduit simultanément ou ultérieurement les azométhines obtenues, ou on réduit des composés répondant à la formule V

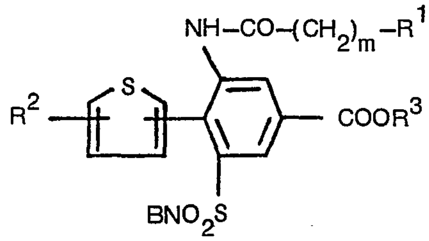

(V)

dans laquelle $R^1$ à $R^3$, B et m ont la signification ci-dessus, avec du diborane ou des borohydrures complexes, le cas échéant en présence d'acides de Lewis, et on hydrolyse ensuite le cas échéant les composés ainsi obtenus et on les transforme le cas échéant en leurs sels par traitement avec des acides ou des bases pharmaceutiquement acceptables.

2. Procédé selon la revendication 1, caractérisé en ce que dans la formule générale I n est égal à 1 et dans les formules générales IV et V m est égal à 0.

3. Procédé selon la revendication 1, caractérisé en ce que l'on prépare l'acide 3-benzylamino-4-(2-thiényl)-5-sulfamoyl-benzoïque.

4. Procédé selon la revendication 1, caractérisé en ce que l'on prépare l'acide 3-benzylamino-4-(3-thiényl)-5-sulfamoyl-benzoïque.

5. procédé selon la revendication 1, caractérisé en ce que l'on prépare l'acide 3-(3'-thénylamino)-4-(2-thiényl)-5-sulfamoyl-benzoïque.

6. Procédé selon la revendication 1, caractérisé en ce que l'on prépare l'acide 3-(2'-thénylamino)-4-(2-thiényl)-5-sulfamoyl-benzoïque.